Europäisches Patentamt

European Patent Office

Office européen des brevets

(19)

(11) Veröffentlichungsnummer: **0 255 651 B1**

(12) **EUROPÄISCHE PATENTSCHRIFT**

(45) Veröffentlichungstag der Patentschrift: **18.03.92**

(21) Anmeldenummer: **87110539.1**

(22) Anmeldetag: **21.07.87**

(51) Int. Cl.⁵: **A61K 37/64**, //(A61K37/64, 37:22,35:16,33:06)

(54) **Mittel zur Therapie faktor VIII-resistenter Hämophilie A und Verfahren zu seiner Herstellung.**

(30) Priorität: **24.07.86 DE 3625090**

(43) Veröffentlichungstag der Anmeldung:
**10.02.88 Patentblatt 88/06**

(45) Bekanntmachung des Hinweises auf die
Patenterteilung:
**18.03.92 Patentblatt 92/12**

(84) Benannte Vertragsstaaten:
**AT BE CH DE ES FR GB GR IT LI LU NL SE**

(56) Entgegenhaltungen:
**EP-A- 0 041 173**
**EP-A- 0 129 998**
**FR-A- 2 330 408**
**FR-A- 2 362 633**

**THROMBOSIS RESEARCH, Nr. 21, 1981, Seiten 181-186, Pergamon Press Ltd, US; T.W. BARROWCLIFFE et al.: "Factor VIII inhibitor bypassing activity: A suggested mechanism of action"**

**JOURNAL OF LABORATORY AND CLINICAL MEDICINE, Band 101, 1983, Seiten 34-43, The C.V. Mosby Co.; T.W. BARROWCLIFFE et al.:**

**"Binding to phospholipid protects factor VIII from inactivation by human antibodies"**

(73) Patentinhaber: **BEHRINGWERKE Aktiengesell-schaft**
**Postfach 1140**
**W-3550 Marburg 1(DE)**

(72) Erfinder: **Heimburger, Norbert, Prof.Dr.**
**Sonnenhang 10**
**W-3550 Marburg(DE)**
Erfinder: **Wenz, Karlheinz**
**Talstrasse 10**
**W-3556 Weimar(DE)**
Erfinder: **Wormsbächer, Wilfried**
**Blumenweg 9**
**W-3575 Kirchhain(DE)**

(74) Vertreter: **Klein, Otto, Dr. et al**
**Hoechst AG Zentrale Patentabteilung Post-fach 80 03 20**
**W-6230 Frankturt am Main 80(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Mittel zur Behandlung von Patienten mit Hämophilie A, die auf die übliche Behandlung mit Faktor VIII nicht ansprechen, sowie ein Verfahren zu seiner Herstellung.

Bis zu etwa einem Viertel der Hämophilie A-Patienten, die mit Faktor VIII-Konzentraten behandelt werden, entwickeln sogenannte Hemmkörper-Hämophilien. Diese sind dadurch gekennzeichnet, daß im Blut des Patienten nicht präzipitierende Isoantikörper gegen die Untereinheit des Faktor VIII-Moleküls, die die Clotting-Aktivität (F VIII:C) trägt, zirkulieren. Diese Antikörper, die bis zu sehr hohen Titern, nämlich von 100 bis zu einigen 1000 E/ml, im Plasma von Hämophilen gefunden werden, neutralisieren entsprechende Aktivitäten von F VIII, wenn diese zur Substitution Patienten infundiert werden, die den Faktor VIII nicht oder nicht in ausreichender Menge synthetisieren. Häufig ist die Menge an inhibierenden Antikörpern so hoch, daß auch die Zufuhr großer Faktor VIII-Mengen nicht zu einem therapeutischen Erfolg führt.

Zur Behandlung der schweren Blutungen, die häufig bei Hämophilie A-Patienten, die Antikörper entwickelt haben, auftreten, wurde eine Reihe von Maßnahmen versucht, die aber nur teilweise zum Erfolg geführt haben. Zu diesen Maßnahmen gehört die Infusion von Prothrombin-Komplex-Konzentraten (PCC), die Faktor II, VII, IX und X enthalten. In Notfallsituationen wurde sogar Faktor VIII aus tierischem Plasma, vorwiegend aus Rinder- oder Schweineplasma, unter Inkaufnahme des Risikos der Zufuhr von Fremdeiweiß verwendet. In jüngerer Zeit wurden auch aktivierte Gerinnungsfaktoren mit einem gewissen Erfolg verwendet. Solche Mittel enthalten aktivierte Gerinnungsenzyme, deren Wirkung schwer steuerbar ist, so daß ein Thromboserisiko nicht ausgeschlossen werden kann.

Man hat auch versucht, diese Antikörper nach dem Prinzip der Immunadsorption an insolubilisierten Faktor VIII aus Patientenplasma zu entfernen. Daneben wird auch der Plasmaaustausch zur Elimination der Antikörper angewandt. Schließlich versucht man auch, die Bildung von Antikörpern gegen F VIII:C therapeutisch zu inhibieren.

In jüngerer Zeit werden auch Komplexe aus F VIII mit Phospholipiden für die Therapie von Hemmkörperhämophilien in Betracht gezogen (J. Lab. Clin. Med. 101, 34-43, 1983). Diese Näherungsweise basiert auf der Erkenntnis, daß der F VIII Phospholipide bindet und es gerade diese Bindungsstellen sind, gegen die die Isoantikörper der Hämophilie-Patienten gerichtet sind. In Übereinstimmung damit sind F VIII-Phospholipid-Komplexe vor dem Angriff von Hemmkörpern vom Typ der Iso-und Auto-Antikörper geschützt. Entsprechende Komplexe werden neben aktivierten Enzymen auch als das aktive Prinzip von FEIBA (Factor VIII Inhibitor Bypass Activity) diskutiert (Thrombosis Research 21, 181-186, 1981).

Die Wirksamkeit von F VIII-Phospholipid-Komplexen ist experimentell gut fundiert. So wird z. B. in J. Lab. Clin. Med. 101, 34-43 (1983) demonstriert, daß ein solcher Komplex, wenn er zusammen mit F IXa einem Hemmkörperplasma zugesetzt wird, die Bildung von Thrombin und damit auch die Gerinnung auslöst, sobald Calcium-Ionen zugegeben werden. Obwohl dieses therapeutische Konzept aussichtsreich erscheint, so hat es doch den Nachteil, daß aktivierte Faktoren beim Produktionsprozeß nur schwer zu handhaben und auch in ihrer in-vivo-Wirkung nicht abzuschätzen sind.

Alle diese Methoden weisen Nachteile auf.

Der vorliegenden Erfindung liegt demnach die Aufgabe zugrunde, ein Mittel zur Behandlung der faktor VIII-resistenten Hämophilie zur Verfügung zu stellen.

Gegenstand der vorliegenden Erfindung ist ein Mittel zur zur Therapie einer gegen eine Behandlung mit Faktor VIII resistenten Hämophilie A, erhältlich dadurch, daß ein Gemisch aus Faktor VIII, Antithrombin III, einem Phospholipid und Calciumionen in einer wässrigen Lösung mindestens 1 Minute bei einer Temperatur von 1 bis 45°C, vorzugsweise 37°C gehalten, Faktor IX zugegeben und die Lösung solange bei einer Temperatur von 1 bis 45°C, vorzugsweise 20°C, gehalten wird, bis eine Probe dieser Lösung einem Hemmkörperplasma zugegeben eine partielle Thromboplastinzeit (PTZ) von 15 bis 30 Sekunden ergibt, gegebenenfalls ein Polyol und gegebenenfalls eine Aminosäure zugegeben und die Lösung gegebenenfalls zur Trockne gebracht wird.

Gegenstand der Erfindung ist ebenfalls das Verfahren zur Herstellung dieses Mittels.

Während der Inkubation entsteht über die Wechselwirkung der Faktoren mit Phospholipiden und Ca-Ionen ein Aktivierungskomplex, der auch in Gegenwart von Isoantikörpern gegen F VIII den endogenen Gerinnungsweg zu aktivieren vermag. Voraussetzung dafür ist, daß die Faktoren im Aktivierungsansatz in einem bestimmten Verhältnis vorliegen, vorzugsweise bezogen auf 4 E F VIII 0,5 - 2 E F IX, 0,5 - 1 E AT III bei mindestens 25 $\mu$g Phospholipid und einer Konzentration von $CaCl_2$ entsprechend 0,75 mmol/l.

Überraschend war, daß das erfindungsgemäße Verfahren zu einem Mittel führt, das frei von amidolytischer und proteolityscher Aktivität ist, wie über die Testung an den chromogenen Substraten S-2238 und S-2222 der Firma Kabi gefunden wurde, und auch Fibrinogen nicht in Fibrin umwandelt. Demnach kann dieses Mittel keine aktivierten Enzyme enthalten. Dazu paßt auch, daß es sich im Aktivierungsansatz in Gegenwart von AT III bildet und durch dieses auch nicht in seiner Wirkung beeinträchtigt wird, wie über die Verkürzung einer durch Hemmkörper pathologisch verlängerten partiellen Thromboplastinzeit (PTZ) augenscheinlich ist.

Das nach diesem Verfahren hergestellte Mittel bleibt über mehrere Tage bei 4°C stabil. Dies ist überraschend, da bekannt ist, daß sich Aktivierungskomplexe, wie sie sich in Form des Faktor X- oder Prothrombinaktivators im Körper bilden, instabil sind.

Überraschend war auch, daß die Wirkung des Mittels gemäß diesem Verfahren verlorengeht, wenn es in wässriger Lösung eingefroren und wieder aufgetaut oder gefriergetrocknet wird. Das Mittel gewinnt jedoch seine Aktivität zurück, wenn es nach Auftauen erwärmt wird, vorzugsweise auf 30 - 37°C. Die Reaktivierung entspricht der Aktivierung gemäß Figur. Im Hinblick auf die Inaktivierung durch Einfrieren und Gefriertrocknung war überraschend, daß durch den Zusatz von Kohlenhydraten, vorzugsweise dem Disaccharid Saccharose, die Inaktivierung verhindert werden konnte.

Überraschend war schließlich auch, daß die Hemmkörperneutralisierende Aktivität des erfindungsgemäßen Mittels nach Trennng an Sepharose CL 6B im Ausschlußpeak erschien. In den die Aktivität enthaltenden Faktoren wurde auch das gesamte zugesetzte Phospholipid gefunden. Mit der Technik des Immunoblottings (nach Trennung in der SDS-Polyacrylamid-Gelelektrophorese) konnten in diesen Fraktionen Derivate des F VIII, F IX, AT III und eine Komponente mit dem Molekulargewicht von 100 kD nachgewiesen werden, die in keinem der zugesetzten Faktorenkonzentrate selbst enthalten war. Aufgrund dieser Befunde ist es naheliegend, daß das erfindungsgemäße Mittel mit einem Komplex aus modifiziertem F VIII und F IX identisch ist, der sich unter dem Schutz von AT III mit Phospholipiden und Ca-Ionen bildet.

Die folgenden Beispiele erläutern die Erfindung.

Materialien, die für die Herstellung und Charakterisierung des erfindungsgemäßen Mittels in den nachfolgenden Beispielen verwendet werden:

Faktor VIII: alle handelsüblichen Faktor VIII-Konzentrate, einschließlich F VIII-Präparationen, auch biotechnischer Herkunft, besonders pasteurisierte Präparate; bevorzugt [R] Haemate P, Faktor VIII-Konzentrat der Behringwerke AG Marburg (Bundesrepublik Deutschland) 50 IE/ml, dialysiert gegen Natrium-Acetat-Puffer, 0,02 mol/l, pH 7,0, enthaltend 0,06 mol/l NaCl und 1 % Glycin;

Faktor IX: Faktor IX-Konzentrat HS Behringwerke, frei von Heparin und frei von Citrat-Ionen durch Dialyse gegen den vorgenannten Natrium-Acetat-Puffer (120 IE/ml F IX);

Phospholipid-Lösung: ein Tetrahydrofuran-Extrakt aus blutfrei gewaschenen und getrockneten Plazenten, 250 mg in 50 ml destilliertem Wasser emulgiert, sterilfiltriert und im Eisbad bei 0°C 20 min. mit 50.000 hz ultrabeschallt, anschließend 1 Stunde bei 100.000 × g bei 4°C zentrifugiert. Es wurde der klare Überstand verwendet. Er enthält ein Gemisch folgender Phospholipide: Phosphatidyläthanolamin (PE), Phosphatidyl-serin (PS), Phosphatidyl-cholin (PC), Phosphatidyl-inositol (PJ), Sphingomyelin (Sph) und Lysoverbindungen (L);

0,1 mol/l wässrige Calciumchlorid-Lösung

Antithrombin III-Lösung:[R] Kybernin HS 500 (Behringwerke AG). 1 Abfüllung mit 500 E bestimmt als Heparin-Cofaktor in 10 ml destilliertem Wasser gelöst

Saccharose: reinst (Merck, Darmstadt, Bundesrepublik Deutschland)

Puffer:

a) 20 mmol/l Na-Acetat, 20 mmol/l NaCl, pH 7,5 (Leitfähigkeit 3,8 mS/cm 25°C)

b) 50 mmol/l Imidazol, 20 mmol/l NaCl, pH 7,5 (Leitfähigkeit 3,7 mS/cm 25°C).

Beispiel 1

1. Herstellung des erfindungsgemäßen Mittels

1,4 ml Faktor VIII-Konzentrat wurden mit 14,5 ml Puffer a verdünnt und 175 µl Antithrombin III-Lösung, 80 µl Phospholipid-Lösung, 120 µl Calciumchlorid-Losung und 0,32 g Saccharose zugegeben und das Gemisch Minuten auf 37°C gehalten. Dann wurde 70 µl Faktor IX-Lösung zupipettiert. Das Gemisch wurde 3 Stunden bei 20°C gehalten, in bestimmten Zeitintervallen (Figur) aliquote Mengen entnommen und durch Bestimmen der partiellen Thromboplastinzeit (PTZ) in einem "Hemmkörperplasma", das heißt einem Plasma, das Antikörper gegen Faktor VIII enthält, geprüft. Als Kontrollen dienten Ansätze, die kein Phospholipid oder kein Calciumchlorid enthielten. Die Prüfung erfolgte in der Weise, daß bei 37°C zu 0,1 ml Hemmkörperplasma mit 80 Bu/ml (Bu = Bethesda units) 0,1 ml des oben hergestellten Gemisches, 0,1 ml eines PTZ-Reagenzes, beispielsweise [R] Pathromtin und 6 min. später 0,1 ml 0,025 mol/l Calciumchlorid-Lösung zugesetzt wurden.

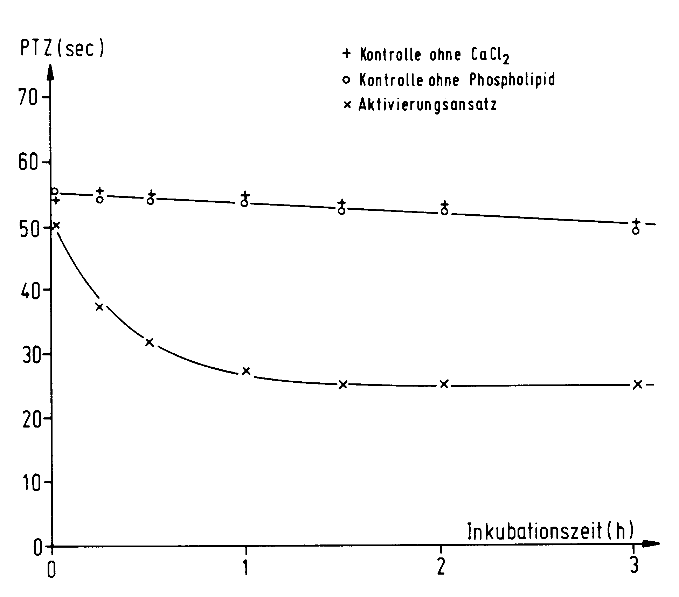

Die Figur enthält die Ergebnisse:

x    erfindungsgemäßes Mittel;
+    dieses Mittel ohne Calciumionen;
o    dieses Mittel ohne Phospholipid

2. Charakterisierung des erfindungsgemäßen Mittels an Hämophilie A-Plasmen mit Hemmkörpern gegen F VIII:C

Das unter 1. hergestellte Mittel wurde an Hemmkörperplasmen mit verschiedenen Titern an Antikörpern geprüft.

In der folgenden Tabelle sind die Ergebnisse dargestellt.

Tabelle 1

| Hemmkörper-Titer | 80 Bu | 160 Bu | 320 Bu | 640 Bu |
|---|---|---|---|---|
| PTZ (sec) | 29,3 29,6 | 30,5 30,5 | 31,3 31,6 | 35,8 35,0 |

Die Ergebnisse lassen erkennen, daß die pathologisch verlängerte PTZ von Hemmkörperplasmen mit Antikörpertitern entsprechend 80 - 640 Bu/ml durch das erfindungsgemäße Mittel normalisiert wird. Sowohl das Hemmkörperplasma mit 80 als auch mit 640 Bu ergab ohne Zusatz eines solchen Mittels PTZ-Werte von etwa 120 Sekunden, die sich auch nach Zugabe von Faktor VIII nicht verkürzen ließen. Daher zeigen die Versuchsergebnisse in Tabelle 1 deutlich, daß das erfindungsgemäße Mittel von den Hemmkörpern nicht abgefangen und neutralisiert wird - im Gegensatz zum Faktor VIII. Daher ist dieses Mittel für die Therapie von Hämophilen mit Hemmkörpern gegen Faktor VIII z. B. vom Typ der Isoantikörper gut geeignet.

Die Wirksamkeit des erfindungsgemäßen Mittels läßt sich auch im Thrombelastographen (TEG) mit Citratvollblut vom Menschen nachweisen.

Wenn Citratvollblut (230 μl) mit Hemmkörper-Plasma (20 μl mit 1400 Bu/ml) gemischt worden war, verlängerte sich erwartungsgemäß die r- und k-Zeit der Thrombelastogramme. Durch Zusatz des erfindungsgemäßen Mittels nach Beispiel 1 (10 μl) wurden die verlängerten r- und k-Werte normalisiert.

3. Stabilität des erfindungsgemäßen Mittels in Citratplasma

Um die Stabilität des erfindungsgemäßen Mittels in Human-Citratplasma zu testen, wurde dieses im Verhältnis 1 + 1 oder 1 + 7 mit Human-Citratplasma inkubiert. Zu bestimmten Zeiten wurden Aliquote von 0,2 ml der Mischung entnommen, mit 0,1 ml PTZ-Reagenz inkubiert und nach 6 min. bei 37°C 0,1 ml 0,025 mol/l $CaCl_2$-Lösung zugegeben. Die nach bestimmten Inkubationszeiten erhaltenen PTZ-Werte sind in der nachfolgenden Tabelle 2 protokolliert.

Tabelle 2

| Stabilität des Aktivator-Komplexes in Citratplasma bei 37°C (Zahlenwerte: PTZ in Sekunden) | | | | | | |
|---|---|---|---|---|---|---|
| | Kontrolle[*] | Inkubationszeiten in Minuten | | | | |
| | | 0-Wert | 7 | 15 | 60 | 150 |
| Mittel + Citratplasma (1:1) | 41,6 41,5 | 21,6 22,1 | 24,4 24,6 | 25,2 25,0 | 26,0 26,1 | 29,5 29,2 |
| Mittel + Citratplasma (1:7) | 40,1 39,1 | 31,4 31,1 | 33,6 33,1 | 34,2 34,2 | 34,9 35,1 | |
| * erfindungsgemäßes Mittel durch Puffer a ersetzt. | | | | | | |

Den Ergebnissen ist zu entnehmen, daß das Mittel in Citratplasma über eine Stunde hinweg stabil ist und auch nach 2 1/2 Stunden noch wirksam ist. Wie die Verkürzung der PTZ gegenüber der Kontrolle mit Puffer zeigt, hat das Mittel eine starke procoagulatorische Wirkung auf Citratplasma. Da die Wirkung auch während der Inkubation mit Citratplasma nicht verlorengeht, kann sie nicht an ein Gerinnungsenzym gebunden sein; denn Citratplasma enthält Antithrombin III, das alle Gerinnungsenzyme neutralisiert.

Beispiel 2

Zur Herstellung des erfindungsgemäßen Mittels wurden
80 ml F VIII-Konzentrat mit
920 ml Puffer b gemischt; daraus resultiert
1000 ml F VIII-Lösung mit 4 E/ml, der folgende Substanzen zugemischt wurden:
10,1 ml AT III-Konzentrat mit 50 E/ml,
5 ml Phospholipid 0,5 %ig (g/100 ml),
7,6 ml 0,1 mol/l $CaCl_2$-Lösung und
20 g Saccharose
Diese Mischung wurde 30 min. bei 37°C inkubiert, dann wurden 4,2 ml F IX-Konzentrat (120 E F IX/ml) zugegeben und 3 Std. bei 20°C inkubiert. Während dieser Zeit hatte sich die in einem Hemmkörper-Plasma damit gemessene PTZ auf 25 sec. verkürzt. Die Lösung wurde sterilfiltriert, in 10 ml-Mengen abgefüllt und lyophilisiert. Dieses Lyophilisat wurde im ursprünglichen Volumen gelöst und eine 4 ml/kg Körpergewicht entsprechende Menge Kaninchen i. v. injiziert. Als Placebo erhielten die Tiere eine Lösung, die analog der oben hergestellt war, jedoch kein Calciumchlorid enthielt. Nach 5, 10, 15 und 30 min. wurde den beiden Tiergruppen, bestehend aus jeweils 3 Kaninchen, Blut entnommen und in dem daraus gewonnenen Plasma die PTZ bestimmt, einmal ohne Zusatz und zum anderen mit Zusatz von Hemmkörperplasma eines Hämophilie A-Patienten. Den tabellarisch zusammengestellten Ergebnissen ist zu entnehmen, daß die PTZ der Kaninchen, die das Placebo erhielten, nur nach 5 min. wenig verkürzt war, aber die PTZ der Tiere, denen das erfindungsgemäße Mittel injiziert wurde (Tabelle 3 a) wesentlich mehr erniedrigt war.

Das Plasma dieser Tiergruppe verkürzte auch die durch Zusatz von Hemmkörpern künstlich verlängerte PTZ (Tabelle 3 b).

Dies belegt, daß das erfindungsgemäße Mittel in aktiver Form im Tier zirkuliert.

Tabelle 3 a

PTZ-Bestimmung ohne Zugabe von Hemmkörperplasma

|  | vor | Applikation | | nach |
|---|---|---|---|---|
|  |  | 5 min | 15 min | 30 min |
| Erfindungsgemäßes Mittel (3 Kaninchen) | 41,7 | 34,7 | 37,9 | 42,2 |
| Placebo (3 Kaninchen) | 42,4 | 38,5 | 46,6 | 42,0 |

PTZ-Bestimmung:  0,1 ml Kaninchenplasma
0,1 ml Diäthylbarbiturat-Acetat-Puffer, pH 7,6
0,1 ml (R) Pathromtin
120 sec 37°C
0,1 ml $CaCl_2$, 0,025 mol/l

Tabelle 3 b

PTZ-Bestimmung unter Zugabe von Hemmkörperplasma

|  | vor | Applikation | | nach |
|---|---|---|---|---|
|  |  | 5 min | 15 min | 30 min |
| Erfindungsgem. Mittel (3 Kaninchen) | 50,2 | 45,1 | 45,2 | 47,2 |
| Placebo (3 Kaninchen) | 61,0 | 60,2 | 61,5 | 61,0 |

PTZ-Bestimmung:  0,1 ml Kaninchenplasma
0,1 ml Hemmkörperplasma 80 Bu/ml
0,1 ml (R) Pathromtin
360 sec 37°C
0,1 ml $CaCl_2$, 0,025 mol/l

Bei Trennung des erfinderischen Mittels an Sepharose CL 6B wurde die gesamte Hemmkörper-neutralisierende Aktivität im Ausschlußvolumen gefunden. In denselben Fraktionen konnten auch die Phospholipide und bei der Analyse der Proben mit der Technik des Immunoblottings unter Verwendung von Antiseren gegen F VIII, F IX und Antithrombin III folgende Komponenten identifiziert werden: neben von-Willebrand-Faktor die modifizierte L-Kette des F VIII:C als Dublette mit Molekulargewichten von 70-75 kD, F IX, Antithrombin III und eine Komponente mit dem Molekulargewicht von 100 kD.

**Patentansprüche**
**Patentansprüche für folgende Vertragsstaaten : BE, CH, DE, FR, GB, IT LI, LU, NL, SE**

1. Mittel zur Therapie einer gegen eine Behandlung mit Faktor VIII resistenten Hämophilie A, erhältlich dadurch, daß ein Gemisch enthaltend auf 4 Einheiten Faktor VIII 0.5 bis 1 Einheit Antithrombin III und mindestens 25 $\mu$g Phospholipid und eine $CaCl_2$-Konzentration von 0.6 - 0.9 mmol/l in einer wässrigen Lösung mindestens 1 Minute bei einer Temperatur von 1 bis 45°C inkubiert wird, 0.5 bis 2 Einheiten Faktor IX zugegeben werden und die Lösung solange bei einer Temperatur von 1 bis 45°C gehalten wird, bis eine Probe dieser Lösung, einem Hemmkörperplasma zugegeben, eine partielle Thromboplastinzeit (PTZ) von 15 bis 30 Sekunden ergibt, daß gegebenenfalls ein Polyol und gegebenenfalls eine Aminosäure zugegeben und gegebenenfalls die Lösung lyophilisiert wird.

2. Mittel nach Anspruch 1, dadurch gekennzeichnet, daß es ein Polyol enthält.

3. Mittel nach Anspruch 1, dadurch gekennzeichnet, daß es eine Aminosäure enthält.

4. Mittel nach Anspruch 1, dadurch gekennzeichnet, daß es lyophilisiert vorliegt.

5. Ein therapeutisches Mittel, hergestellt nach Anspruch 1 und wie folgt gekennzeichnet:
   - starke prokoagulatorische Wirkung aber frei von aktivierten Gerinnungsenzymen nach Testung an chromogenen Substraten für F IIa und Xa;
   - stabil in humanem Citratplasma;
   - verkürzt die PTZ von normalem Human-Citratplasma und von
   - Plasma mit Hemmkörpern gegen F VIII:C;
   - Wirkung wird durch Antikörper gegen F IX nicht beeinträchtigt;
   - Nach Gelfiltration an Sepharose CL 6B wandert die Aktivität im Ausschlußvolumen und die Fraktionen mit Hemmkörperaktivität enthalten Phospholipide, von-Willebrand-Antigen, die modifizierte L-Kette des F VIII:C (Molekulargewicht 70-75 000), F IX eine Komponente mit einem Molekulargewicht vo 100 kD und Antithrombin III.

**Patentansprüche für folgende Vertragsstaaten : GR, ES, AT**

1. Verfahren zur Herstellung eines Mittels zur Therapie einer gegen eine Behandlung mit Faktor VIII resistenten Hämophilie A, gekennzeichnet dadurch, daß ein Gemisch enthaltend auf 4 Einheiten Faktor VIII 0.5 bis 1 Einheit Antithrombin III und mindestens 25 $\mu$g Phospholipid und eine $CaCl_2$-Konzentration von 0.6 - 0.9 mmol/l in einer wässrigen Lösung mindestens 1 Minute bei einer Temperatur von 1 bis 45°C inkubiert wird, 0.5 bis 2 Einheiten Faktor IX zugegeben werden und die Lösung solange bei einer Temperatur von 1 bis 45°C gehalten wird, bis eine Probe dieser Lösung, einem Hemmkörperplasma zugegeben, eine partielle Thromboplastinzeit (PTZ) von 15 bis 30 Sekunden ergibt, daß gegebenenfalls ein Polyol und gegebenenfalls eine Aminosäure zugegeben und gegebenenfalls die Lösung lyophilisiert wird.

2. Verfahren nach Anspruch 1, dadurch gekennzeichnet, daß ein Polyol zugegeben wird.

3. Verfahren nach Anspruch 1, dadurch gekennzeichnet, daß eine Aminosäure zugegeben wird.

4. Verfahren nach Anspruch 1, dadurch gekennzeichnet, daß lyophilisiert wird.

**Claims**
**Claims for the following Contracting States : BE, CH, DE, FR, GB, IT, LI, LU, NL, SE**

1. An agent for the therapy of hemophilia A which is resistant to treatment with factor VIII, obtainable by incubating a mixture containing per 4 units of factor VIII 0.5 to 1 unit of antithrombin III and at least 25 $\mu$g of phospholipid and a $CaCl_2$ concentration of 0.6 - 0.9 mmol/l in an aqueous solution at a temperature of from 1 to 45°C for at least one minute, adding 0.5 to 2 units of factor IX, and maintaining the solution at a temperature of from 1 to 45°C until addition of a sample of this solution to an inhibitor plasma results in a partial thromboplastin time (PTT) of 15 to 30 seconds, where appropriate adding a polyol and, where appropriate, an amino acid, and, where appropriate, freeze-drying the solution.

2. An agent as claimed in claim 1, which contains a polyol.

3. An agent as claimed in claim 1, which contains an amino acid.

4. An agent as claimed in claim 1, which is in freeze-dried form.

5. A therapeutic agent prepared as claimed in claim 1 and having the following characteristics:
potent procoagulant action but containing no activated coagulation enzymes as shown by testing on chromogenic substrates for F 11a and Xa; stable in citrated human plasma;
reduces the PTT of normal citrated human plasma and of
plasma containing inhibitors of F VIII:C;
effect is not impaired by antibodies against F IX;
on gel filtration on Sepharose CL 6B, the activity migrates in the exclusion volume, and the fractions with inhibitory activity contain phospholipids, von Willebrand antigen, the modified L chain of F VIII:C (molecular weight 70-75,000), F IX, a component having a molecular weight of 100 kD and antithrombin III.

**Claims for the following Contracting States : GR, ES, AT**

1. A process for the preparation of an agent for the therapy of hemophilia A which is resistant to treatment with factor VIII, which comprises incubating a mixture containing per 4 units of factor VIII 0.5 to 1 unit of antithrombin III and at least 25 $\mu$g of phospholipid and a $CaCl_2$ concentration of 0.6 - 0.9 mmol/l in an aqueous solution at a temperature of from 1 to 45°C for at least one minute, adding 0.5 to 2 units of factor IX, and maintaining the solution at a temperature of from 1 to 45°C until addition of a sample of this solution to an inhibitor plasma results in a partial thromboplastin time (PTT) of 15 to 30 seconds, where appropriate adding a polyol and, where appropriate, an amino acid, and, where appropriate, freeze-drying the solution.

2. The process as claimed in claim 1, wherein a polyol is added.

3. The process as claimed in claim 1, wherein an amino acid is added.

4. The process as claimed in claim 1, wherein freeze-drying takes place.

**Revendications**
**Revendications pour les Etats contractants suivants : BE, CH, DE, FR, GB, IT, LI, LU, NL, SE**

1. Agent pour le traitement thérapeutique d'une hémophilie A résistante au traitement avec le facteur VIII, obtenu par: incubation pendant au moins 1 minute, à une température de 1 à 45°C, d'un mélange contenant, pour 4 unités de facteur VIII, 0,5 à 1 unité d'antithrombine III et au moins 25 $\mu$g de phospholipide ainsi que du $CaCl_2$ à une concentration de 0,6 à 0,9 mole/l en solution aqueuse, addition de 0,5 à 2 unités de facteur IX, maintien de la solution obtenue à une température de 1 à 45°C aussi longtemps qu'il le faut pour qu'un échantillon de cette solution, ajouté à un plasma renfermant des inhibiteurs d'immunité, présente un temps partiel de thromboplastine (PTT) de 15 à 30 secondes, addition éventuelle d'un polyol et éventuellement d'un acide aminé, et, éventuellement, lyophilisation de la solution.

2. Agent selon la revendication 1, caractérisé en ce qu'il contient un polyol.

3. Agent selon la revendication 1, caractérisé en ce qu'il contient un acide aminé.

4. Agent selon la revendication 1, caractérisé en ce qu'il se présente lyophilisé.

5. Agent thérapeutique, préparé selon la revendication 1 et caractérisé comme suit:

- forte action procoagulante, mais sans enzymes de coagulation activées d'après l'essai sur substrats chromogènes pour F IIa et Xa,
- stable dans le plasma citraté humain,
- raccourcit le PTT du plasma citraté humain et du
- plasma renfermant des inhibiteurs contre F VIII:C,
- son action n'est pas affectée par les anticorps contre F IX,
- après filtration sur gel de Sepharose CL 6B, l'activité migre dans le volume d'exclusion, et les fractions dotées d'activité inhibitrice renferment les phospholipides, l'antigène von Willebrand, la chaîne L modifiée de F VIII:C (masse moléculaire 70-75000), F IX, un composant de masse moléculaire 100 kD et l'antithrombine III.

**Revendications pour les Etats contractants suivants : GR, ES, AT**

1. Procédé de préparation d'un agent pour le traitement thérapeutique d'une hémophilie A résistante au traitement avec le facteur VIII, caractérisé en ce qu'on fait incuber pendant au moins 1 minute, à une température de 1 à 45°C, un mélange contenant, pour 4 unités de facteur VIII, 0,5 à 1 unité d'antithrombine III et au moins 25 $\mu$g de phospholipide ainsi que du $CaCl_2$ à une concentration de 0,6 à 0,9 mmole/l en solution aqueuse, on y ajoute de 0,5 à 2 unités de facteur IX, on maintient la solution obtenue à une température de 1 à 45°C aussi longtemps qu'il le faut pour qu'un échantillon de cette solution, ajouté à un plasma renfermant des inhibiteurs d'immunité, présente un temps partiel de thromboplastine (PTT) de 15 à 30 secondes, on ajoute éventuellement un polyol et éventuellement un acide aminé, et on lyophilise éventuellement la solution.

2. Procédé selon la revendication 1, caractérisé en ce qu'on ajoute un polyol.

3. Procédé selon la revendication 1, caractérisé en ce qu'on ajoute un acide aminé.

4. Procédé selon la revendication 1, caractérisé en ce qu'on procède à une lyophilisation.